# EUROPEAN PATENT APPLICATION

(11) **EP 4 120 280 A1**
(43) Date of publication of application: **18.01.2023**
(21) Application number: 22150856.7
(22) Date of filing: 11.01.2022
(51) Int. Cl.: G16H 10/60, G16H 20/10, G16H 50/20, A61B 5/00, A61Q 19/00, G06Q 30/06

(54) **CUSTOMER-TAILORED BIG DATA ANALYSIS SYSTEM USING COSMETIC INFORMATION AND BIOMETRIC INFORMATION**

(30) Priority: 16.07.2021 KR 20210093209
(71) Applicant: Saerom Biotech Co., Ltd., Incheon 22376 (KR)
(72) Inventor: PARK, Saerom, 10056 Gimpo-si, Gyeonggi-do (KR)
(74) Representative: M. Zardi & Co S.A.

(57) **Abstract**

A customer-customized big data analysis system using cosmetic information and biometric information according to the present invention, the big data analysis system comprises: a skin care device used by each customer, generating a microcurrent to increase penetration of cosmetics, and detecting biometric information related to skin; a customer terminal connected to the skin care device through short-distance communication, input member information through an application, and displaying the biometric information; and a big data server including a member biometric information DB unit connected to customer terminals through long-distance communication, and receiving and storing member information and biometric information from the customer terminals, and a cosmetics DB unit storing information of cosmetics and additives, wherein the application recommends cosmetics by analyzing big data with its own algorithm.

## Description

### FIELD OF THE INVENTION

The present invention relates to a customer-tailored big data analysis system using cosmetic information and biometric information, and more particularly, to a big data analysis system that can identify customer-tailored cosmetics by using artificial intelligence (Al) algorithms and big data analysis of biometric information obtained from customers using beauty equipment and cosmetic information input by customers or managers.

### BACKGROUND OF THE INVENTION

With the passage of time, the desire to have clean and healthy skin is increasing regardless of age or gender. Accordingly, cosmetic companies are releasing various cosmetics to satisfy these needs, and cosmetics specialized in specific functions, such as skin nourishment, keratin improvement, skin moisturizing, oil-water balance control, elasticity improvement, wrinkle improvement, skin lightening, and UV protection, are being developed.

The primary causes of skin aging can be largely divided into lack of moisture in the skin, active oxygen, and ultraviolet radiation. Lack of moisture causes wrinkles and excessive dead skin cells, reduces elasticity of the skin, and causes various skin problems such as acne. In addition, active oxygen accelerates aging by oxidizing collagen. Furthermore, ultraviolet rays destroy the elastic fibers of the skin, causing wrinkles, darkening the skin tone such as pigmentation, and causing various skin problems.

Therefore, people are using various functional cosmetics to prevent skin aging. However, general cosmetics include dozens to hundreds of different types of additives. Even if vegetable or eco-friendly additives are used to produce cosmetics, chemical additives are also necessarily included in the process of refining. Various chemical additives are also required for function of improving skin.

Cosmetic companies are releasing cosmetics after testing of harmful ingredients in cosmetics through research institutes. However, even in this case, the cosmetics are not safe for all users, and some users may have skin problems with the cosmetics.

Many customers choose cosmetics because they are expensive products, or they like the advertisements of cosmetic, or they have good scents they like. That is, customers cannot consider whether cosmetic additives and the skin of the user fit well. Accordingly, some customers try a sample first before purchasing cosmetics.

However, even after using cosmetics, customers cannot objectively determine the degree of skin improvement, and simply have a subjective judgment based on the occurrence of skin problems, the skin tone visible with the naked eye, and the skin texture after applying the cosmetics.

Meanwhile, some customers use skin care devices together with cosmetics. These devices are designed to generate low-frequency, ultrasonic waves, heat, and negative ions, to clean and remove dead skin cells by operating a brush, or to have a skin massage function.

However, these skin care devices also have limitations that users only obtain their own subjective evaluation on the improvement of the skin conditions, and it is impossible for the users to know the objective efficacy of the device. In addition, since users do not know the degree of the skin improvement after the use of the device, it is impossible to perform customized skin treatment.

### Prior Art

(Patent Document 0001) Korean Patent Publication No. 10-2021-0069259
(Patent Document 0002) Korean Patent Registration No. 10-2058733
(Patent Document 0003) Korean Patent Registration No. 10-1899843

### Technical Problem

The present invention has been devised to solve the above problems, and an object of the present invention is to provide a customer-tailored big data analysis system using cosmetic information and biometric information that can significantly improve the satisfaction of customers in choosing cosmetics and objectively identify the degree of improvement according to the use of cosmetics and skin care devices, by storing biometric information obtained from each customer through their beauty devices and ingredient information of various cosmetics, and analyzing both big data to select customized cosmetics suitable for each customer.

### SUMMARY OF THE INVENTION

A customer-customized big data analysis system using cosmetic information and biometric information comprises: a skin care device individually used by each customer, generating a microcurrent to increase penetration of cosmetics, and detecting biometric information related to a customer's skin information through a sensor; a customer terminal connected to the skin care device through short-distance communication, input member information through a pre-installed application, and displaying the biometric information provided from the skin care device; and a big data server including a member biometric information DB unit connected to a plurality of customer terminals through long-distance communication, and receiving and storing member information and biometric information from the customer terminals, and a cosmetics DB unit storing information on types of cosmetics and additives included in the cosmetics, wherein the application of the customer terminal recommends one or more types of cosmetics included in the cosmetics DB unit, by analyzing big data including the information of the member biometric information DB unit and the information of the cosmetics DB unit 500, using its own algorithm.

In addition, the biometric information displayed on the customer terminal may include information related to moisture content, oil content, skin elasticity, skin aging, and lightness of the skin.

Furthermore, the member information input through the customer terminal may include member ID, age, gender, occupational group, skin type, living environment, disease, and eating habits.

Moreover, the application of the customer terminal may include: a before use record unit recording skin condition before use of cosmetics and the skin care device; an after use record unit recording skin condition after use of the cosmetics and the skin care device, and a comparison and determination unit analyzing and determining a degree of skin improvement by comparing and analyzing data of the before use record unit and the after use record unit.

In this case, the information related to the degree of skin improvement of the comparison and determination unit may be stored in the member biometric information DB unit of the big data server.

In addition, the information of the additives stored in the cosmetic DB unit may be automatically uploaded by recognizing an ingredient QR code of a cosmetic through the customer terminal, or by recognizing the cosmetic QR code and self-searching an ingredient list of the cosmetic through search algorithm of a website in the big data server.

### Advantageous Effects

The customer-tailored big data analysis system of the present invention can provide customized cosmetics use guidance for each customer, help customers select cosmetics suitable for them, and greatly improve the customer's satisfaction in selecting the cosmetics, by storing customer's biometric information, member information, and cosmetic information as big data and repeatedly learning the correlation between both data.

In addition, the customer-tailored big data analysis system has effects of objectively identifying the improvement of cosmetics and improving system reliability, by displaying biometric information related to the skin of customer on the customer terminal through a sensor provided in the skin care device.

Furthermore, since the customer-tailored big data analysis system of the present invention uses biometric information provided by the skin care device without any human intervention, it is possible to have objective and accurate judgment in evaluating each cosmetic. In addition, as the data accumulate, the accuracy can be further improved. This effect can lead to an increase in use and consumption of the skin care device.

### BRIEF DESCRIPTION OF THE DRAWINGS

Fig. 1 is a drawing illustrating an overall configuration of a customer-tailored big data analysis system using cosmetic information and biometric information according to the present invention.

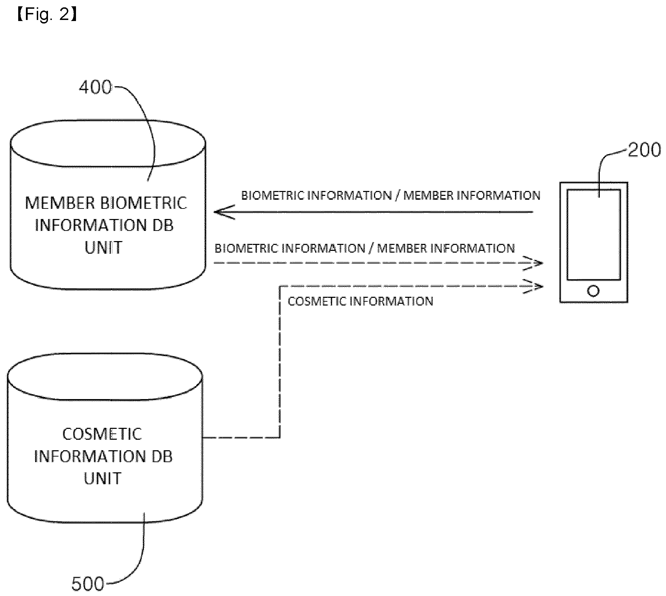

Fig. 2 is an exemplary drawing illustrating information exchanged between a customer terminal and a big data server in the system according to the present invention.
Fig. 3 is a drawing illustrating a configuration identifying the degree of improvement of the skin on a customer terminal application in the system according to the present invention.

### DETAILED DESCIPTION EMBODIEMENTS OF THE INVENTIONS

Hereinafter, preferred embodiments of the present invention will be described in detail with reference to the accompanying drawings. In the description of the present invention, if a detailed description of related known functions or configurations may unnecessarily obscure the gist of the present invention, the detailed description thereof will be omitted.

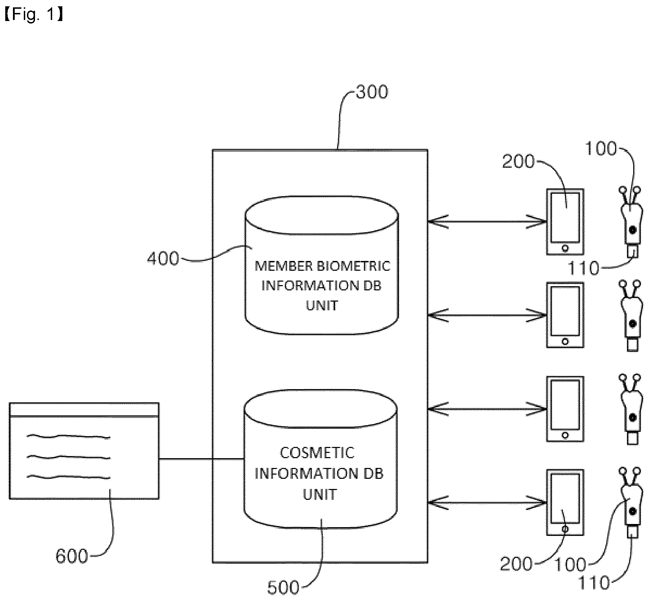

Referring to FIG. 1, a customer-customized big data analysis system using cosmetic information and biometric information of the present invention comprises: a skin care device 100 individually used by each customer, generating a microcurrent to increase penetration of cosmetics, and detecting biometric information related to a customer's skin information through a sensor 110; a customer terminal 200 connected to the skin care device 100 through short-distance communication, input member information through a pre-installed application, and displaying biometric information provided from the skin care device 100; and a big data server 300 including a member biometric information DB unit 400 connected to a plurality of customer terminals 200 through long-distance communication, and receiving and storing member information and biometric information from the customer terminals 200, and a cosmetics DB unit 500 storing information on types of cosmetics and additives included in the cosmetics.

The application of the customer terminal 200 recommends one or more types of cosmetics included in the cosmetics DB unit 500, by analyzing the big data including the information of the member biometric information DB unit 400 and the information of the cosmetics DB unit 500 through the application of the customer terminal 200, using its own algorithm.

The skin care device 100 of the present invention is used by a customer, and generates a microcurrent in a state where cosmetics are applied to the skin, to stimulate the internal cellular tissue of the skin, increase the elasticity of the tissue, and ionize the skin to help the cosmetics to penetrate deep into the skin, thereby increasing the absorption rate of cosmetics.

The skin care device 100 detects in real time biometric information related to the skin information of the customer through the sensor 110 installed therein. The detected information is related to moisture content, oil content, skin elasticity, skin aging, and lightness of the skin.

The customer terminal 200 of the present invention is connected to the skin care device 100 using short-distance communication such as Bluetooth. The biometric information measured by the skin care device 100 is displayed on the screen of the customer terminal 200 through the application installed in the customer terminal 200, so that the customer can directly check the condition of the skin. In this case, the biometric information displayed on the customer terminal 200 includes information related to moisture content, oil content, skin elasticity, skin aging, and lightness of the skin.

In addition, the member information is input through the application of the customer terminal 200. The member information input through the customer terminal 200 may include information of member ID, age, gender, occupational group, skin type, living environment, disease, and eating habits.

The big data server 300 of the present invention is connected to a plurality of customer terminals 200 through long-distance communication such as internet, and member information and biometric information provided from each customer terminal 200 are stored in the member biometric DB unit 400, and types of cosmetics and information on additives included in cosmetics are stored in the cosmetics DB unit 500.

The big data server 300 provides the information of the member biometric DB unit and the cosmetic DB unit 500 to the customer terminal 200, and the application of the customer terminal 200 analyzes big data including the provided information to determine the most suitable cosmetics for the customer to recommends the certain type of cosmetics and displays them on the customer terminal 200.

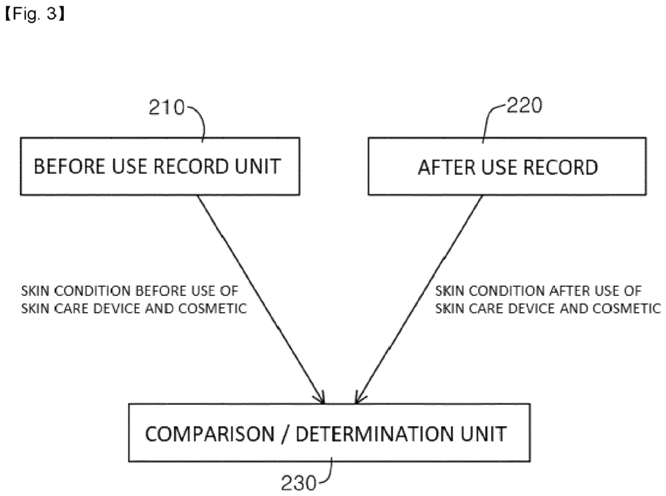

As shown in FIG. 3, the application of the customer terminal 200 may include a before use record unit 210 recording the skin condition before use of cosmetics and the skin care device 100; an after use record unit 220 recording the skin condition after use of the cosmetics and the skin care device 100, and a comparison and determination unit 230 to analyze and determine the degree of skin improvement by comparing and analyzing the data of the before use record unit 210 and the after use record unit 220. When the skin condition before use and the skin condition after use are compared and the degree of skin improvement is displayed on the customer terminal 200, the customer can objectively identify the effect of the skin care device 100 and cosmetics.

Meanwhile, the information related to the degree of skin improvement of the comparison and determination unit 230 is stored in the member biometric information DB unit 400 of the big data server 300.

When the data related to the degree of skin improvement is accumulated in a large amount in the big data server 300, more accurate, customer-tailored cosmetics can be recommended. Specifically, the system analyzes the additives of the certain cosmetic groups by which the level of skin improvement improves, identifies common additives included in the certain cosmetic groups, and recommends other types of cosmetics containing a large amount of the corresponding additives.

On the other hand, if the level of skin improvement deteriorates by using certain cosmetics, the additives contained in those cosmetics are analyzed and excluded specific cosmetics containing the same or similar ingredients from the list of recommendations, and provides the customer with information of specific cosmetics that are required not to use.

Accordingly, the present invention can predict the skin condition of the members and recommend suitable, customized cosmetics for each customer without using cosmetics, by analyzing the correlation with biometric information based on the information of members including age, gender, occupational group, skin type, living environment, disease, and eating habits, and guiding a customer who install the application for the first time to simply enter member information.

For example, if information such as 42 years old, male, field worker, or dry skin type is entered as member information, the system finds other customers who have similar conditions, and then recommends certain cosmetics that those customers use, or other cosmetics that have significant skin improvement to those customers, or have specific additives similar to those included in the cosmetics used by the existing customer.

Meanwhile, the information of additives stored in the cosmetic DB unit 500 is not directly input by a server manager. The information of additives is automatically uploaded by recognizing the ingredient QR code of the cosmetic through the customer terminal 200, or by recognizing the cosmetic QR code and self-searching the ingredient list of the cosmetic through search algorithm of the website 600 in the server. In this case, the additive Information on the website 600 may be obtained by automatically extracting data existing on the website 600 through a crawling program installed in the application of the customer terminal 200.

As described above, since the user directly updates the cosmetics DB unit 500 through the customer terminal 200, the manager does not need to input cosmetic information one by one, and as the number of customers increases, cosmetic information can be accumulated in the cosmetics DB unit 500.

The customer-tailored big data analysis system of the present invention can provide customized cosmetics use guidance for each customer and help customers find cosmetics suitable for them, by storing customer's biometric information, member information, and cosmetic information as big data and repeatedly learning the correlation between both data.

In addition, since the customer-tailored big data analysis system of the present invention uses biometric information provided by the skin care device without any human intervention, it is possible to have objective and accurate judgment in evaluating each cosmetic. In addition, as the data accumulate, the accuracy can be further improved.

Meanwhile, when any one of the cosmetics recommended through the application of the customer terminal 200 is selected and purchased, the system operator may be automatically paid the advertising cost from the manufacturer or seller of the corresponding cosmetics. Accordingly, with this operation, the system of the present invention can activate the cosmetics market.

Although the present invention has been described above with reference to the above embodiments, various modifications are possible within the scope of the technical spirit of the present invention.

## Claims

1. A customer-customized big data analysis system using cosmetic information and biometric information comprises:
a skin care device 100 individually used by each customer, generating a microcurrent to increase penetration of cosmetics, and detecting biometric information related to a customer's skin information through a sensor 110;
a customer terminal 200 connected to the skin care device 100 through short-distance communication, input member information through a pre-installed application, and displaying the biometric information provided from the skin care device 100; and
a big data server 300 including:
a member biometric information DB unit 400 connected to a plurality of customer terminals 200 through long-distance communication, and receiving and storing member information and biometric information from the customer terminals 200, and
a cosmetics DB unit 500 storing information on types of cosmetics and additives included in the cosmetics,
wherein the application of the customer terminal 200 recommends one or more types of cosmetics included in the cosmetics DB unit 500, by analyzing big data including the information of the member biometric information DB unit 400 and the information of the cosmetics DB unit 500 with its own algorithm.

2. The customer-customized big data analysis system of claim 1, wherein the biometric information displayed on the customer terminal 200 includes information related to moisture content, oil content, skin elasticity, skin aging, and lightness of the skin.

3. The customer-customized big data analysis system of claim 1, wherein the member information input through the customer terminal 200 includes member ID, age, gender, occupational group, skin type, living environment, disease, and eating habits.

4. The customer-customized big data analysis system of claim 1, wherein the application of the customer terminal 200 includes:
a before use record unit 210 recording skin condition before use of cosmetics and the skin care device 100;
an after use record unit 220 recording skin condition after use of the cosmetics and the skin care device 100, and
a comparison and determination unit 230 analyzing and determining a degree of skin improvement by comparing and analyzing data of the before use record unit 210 and the after use record unit 220.

5. The customer-customized big data analysis system of claim 4, wherein the information related to the degree of skin improvement of the comparison and determination unit 230 is stored in the member biometric information DB unit 400 of the big data server 300.

6. The customer-customized big data analysis system of claim 1, wherein the information of the additives stored in the cosmetic DB unit 500 is automatically uploaded by recognizing an ingredient QR code of a cosmetic through the customer terminal 200, or by recognizing the cosmetic QR code and self-searching an ingredient list of the cosmetic through search algorithm of a website 600 in the big data server 300.
